# EUROPEAN PATENT SPECIFICATION

(11) **EP 1 370 327 B1**
(45) Date of publication and mention of the grant of the patent: **27.02.2008**
(21) Application number: 02733126.3
(22) Date of filing: 15.03.2002
(51) Int. Cl.: A61N 1/39

(54) **BATTERY PACK FOR AN AUTOMATIC EXTERNAL DEFIBRILLATOR**
BATTERIEANORDNUNG FÜR EINEN AUTOMATISCHEN EXTERNEN DEFIBRILLATOR
BLOC-BATTERIE POUR DEFIBRILLATEUR EXTERNE AUTOMATISE

(30) Priority: 15.03.2001 IE 20010252
(43) Date of publication of application: 17.12.2003
(73) Proprietor: Heartsine Technologies Limited, Belfast BT3 9DU (GB)
(72) Inventor: ALLEN, James, Ballyclare County Antrim BT39 OBS (GB); ANDERSON, Johnny, Houston, County Down BT18 OPJ (GB); ANDERSON, John, McCune, County Down BT18 0NG (GB)
(74) Representative: Casey, Lindsay Joseph
(86) International application number: PCT/IB2002/002025
(87) International publication number: WO 2002/074382

(56) References cited:
- EP-A- 0 743 531
- EP-A- 0 923 961
- EP-A- 1 072 285
- US-A- 5 662 690
- US-A- 5 683 423
- US-A- 5 749 902
- US-A- 5 919 212

## Description

This invention relates to an automatic external defibrillator and a replaceable battery pack therefor.

### Background

Modern automatic external defibrillator (AED) devices are compact portable units which automatically analyse ECG (ElectroCardioGraph) rhythms obtained from the human thorax via electrodes in contact with the skin. These devices are intended to provide a first response to cardiac arrest emergencies by being made available to emergency services and with suitable training to large institutions (shopping malls, casino's, railway stations, airports, etc ). There is also clinical and public pressure to make them available to the general public. Since these devices need to be small, lightweight and easy-to-use they are typically almost completely automatic. Essentially, assuming that the unit is functional and has a battery pack with sufficient charge to operate the unit, then a user attempting to respond to such a cardiac emergency would simply press a "ON" button on the unit and then follow voice and text prompts generated by the device.

A typical sequence of events would be for the device to prompt the user to apply the electrode pads to the patient and make sure that the electrodes are correctly connected to the device. Once the device is satisfied that this has been performed, the device will then automatically analyse the patient's heart rhythm and make a diagnosis. If it determines a high voltage electric shock is warranted, then it will automatically charge to a predetermined energy level and advise the user to press a clearly marked "SHOCK" button. Whether or not the device advises shock, the user is informed continuously about the state of the patient and/or the actions and states of the device.

As part of the safety requirements of such devices, a record of events, decisions and actions must be recorded. Previous to AED emergence, this was accomplished by a paper strip recorder that continuously printed the patient's ECG and annotated the various defibrillator events such as analyse, charge, shock, etc., with time stamps along the top of the paper strip.

### Prior art

In order to keep weight and size to a minimum, currently available AEDs employ digital storage devices, such as memory cards, to store defibrillator events and patient recordings. The use of such storage devices means that a microcontroller or other digital processing system digitises and stores the occurrence of events that need recording and also typically digitises and stores the ECG that has been acquired from the patient's thorax. Some systems also digitise and store the audio activity within the near vicinity of the device. This then allows the sequence of events which occurred as a result of the use of the device, the ECG of the patient over the period of time the device was used and also anything said by the operator, to be stored in the digital memory. Later the digitally stored data can be downloaded to an external computer system to facilitate playback. Alternatively some systems allow the actual AED itself to play back some of the recorded information.

The method of downloading the stored information to an external computer system can be accomplished in various ways. Some systems transfer the information from the AED's digital storage device via an infrared or audio modem link. Where removable storage is used, such as a memory card interfacing with the AED via a PCMCIA interface, the storage device is physically removed from the AED and then plugged into the external computer system where the digitised information can be retrieved.

All of these mechanisms have to solve several problems.
1. It is necessary that all the information stored in the memory card or other digital storage medium can be time referenced. For instance, in one known device the memory card starts a counter, powered by a small watch type battery, from the instant the AED is powered on. This counter keeps running until the memory card is plugged into the external computer. This means that the external computer can retrospectively calculate the actual timing of events by taking the current time and the count reached for a specific event, then count back in time to work out the actual time of the event.
2. Secondly, AEDs have certain features, which may or may not be activated by the institution purchasing them. They also have different modes of operation. For instance, they are capable of being placed in a training mode where the high voltage shock delivery is disabled to prevent harm or damage to the operator or bystanders when training on resuscitation training aids. One known device uses a special memory card storage device containing special codes which is plugged into the AED and the AED therefore recognises that it must perform in a specific training mode.
3. AED status and usage lifetime are also required to be monitored so that service break-points for the whole AED system can be tracked. These devices also automatically perform self-diagnostic checks to maintain a state of readiness and the results from these checks need to be logged and made available to service personnel. They therefore require some form of two-way communication with an external computer system either for downloading of patient/device data, programming of device features/modes and/or time and date synchronisation.
4. AEDs are used in a wide variety of situations and environmental surroundings. They therefore need to be robust and have a certain degree of resistance to water and liquid ingress. Any method of transfer of data therefore needs to be accomplished without compromising the seals of the AED case. One method of accomplishing this has already been mentioned where an infrared or audio modem link is used to communicate with an external system.

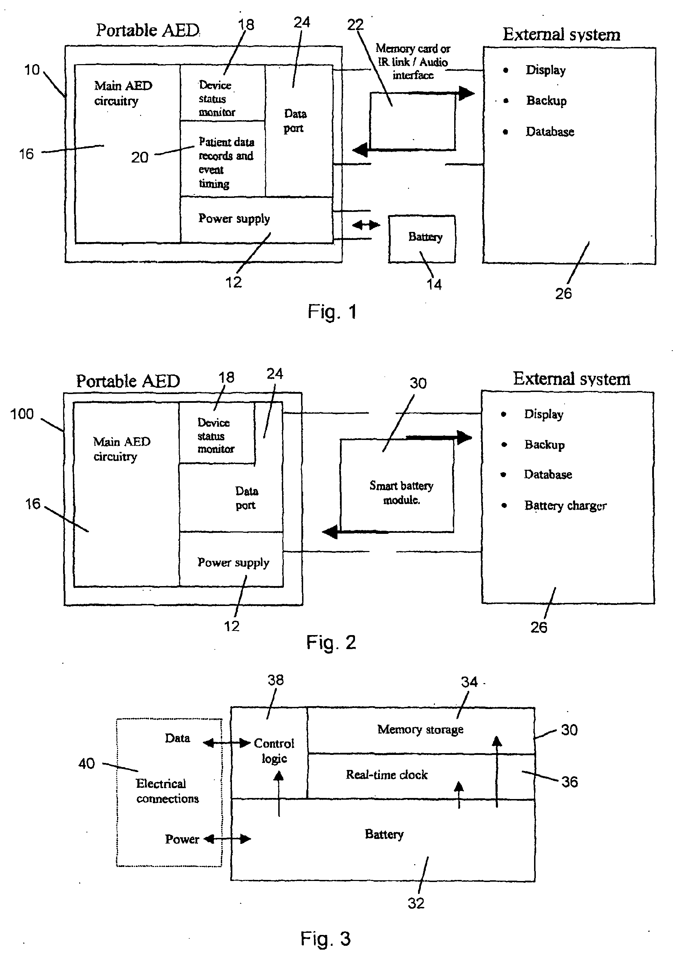

Currently available AEDs solve these problems and requirements in various forms and degrees of satisfaction. Figure 1 shows a typical device structure. The AED 10 contains circuitry 12 to provide power to the device via a plug-in battery pack 14. The device itself contains microprocessor-controlled main circuitry 16 to facilitate the necessary AED functions. A device status monitor 18 is responsible for storing device actions, responses and events to an internal digital storage medium 20 or to a plug-in memory card 22 via a data port 24 (it will be understood that the storage medium 20 and plug-in card 22 are alternatives even though both are shown in Fig. 1). In the case of internal storage the data is transferred to an external system 26 by, for example, an infrared or audio modem link as mentioned above. In the case of a plug-in memory card 22 the data is transferred to the external system by unplugging the card from the AED 10 and plugging it into the system 26.

European Patent Specification no EP-A-0 923 961 considered as closest prior art discloses a battery management system preferably having a base station utilized in connection with a portable electronic device for providing electrical therapy to the body of a patient in response to the occurrence of a treatable condition. The portable device can have a rechargeable battery, memory, data processor for determining available operating time for the portable device prior to recharging, and a display panel, or alarm, to inform the patient of such available operating time. The portable device data processor contains an analog to digital converter which is used to obtain and record data regarding the patient, the battery, and the portable device operational status.

It is an object of the present invention to provide an improved automatic external defibrillator.

### Summary of the invention

According to an aspect of the present invention, there is provided a replaceable battery pack as specified in claim 1. According to another aspect of the present invention, there is provided an automatic external defibrillator as specified in claim 8.

### Brief Description of the Drawings

An embodiment of the invention will now be described, by way of example, with reference to the accompanying drawings, in which:
Fig. 1, previously described, is a schematic drawing of a known automatic external defibrillator;
Fig. 2 is a schematic drawing of an embodiment of an automatic external defibrillator according to the invention;
Fig. 3 is a schematic drawing of the architecture of the battery pack used in the defibrillator of Fig. 2; and

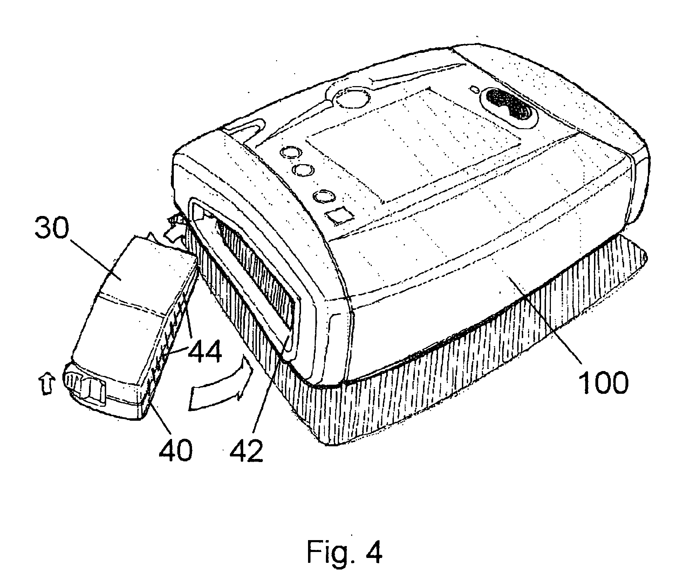

Fig. 4 is a perspective view of the exterior of the defibrillator of Figs. 2 and 3.

### Description of the preferred Embodiment

In Fig. 2 components which are the same or equivalent to those of Fig. 1 have been given the same reference numerals and will not be described again except where necessary to point out any differences.

Referring to Fig. 2 and 3, in the embodiment the patient data, event storage and event timings are no longer an integral part of the AED 100 itself. Instead, they are contained in a battery pack 30 (hereinafter referred to as a Smart Battery Module, or SBM) which provides power to the AED's power supply circuitry 12, storage of defibrillator events, storage of ECG and/or audio data, identification of device mode/settings, time synchronisation, ability to 'wake-up' the AED and instruct it to perform self test protocols at intervals set by the time circuitry of the SBM then store the results of the tests within the SBM, and also the facility to provide a training mode whereby example cardiac events are stored within the SBM for use by the AED for operator training. Fig. 3 shows the internal architecture of a typical embodiment of the SBM 30.

The SBM 30 is a battery pack, i.e. a single self-contained unit or module, which in addition to the battery proper 32 also possesses digital memory 34, a real-time clock 36, control logic 38 to provide intelligent management of the AED 100 and the SBM 30 itself, and a connecting mechanism 40 to provide electrical data and power connections to the AED 100 when the SMB is inserted in an operative position therein. In this embodiment the connecting mechanism 40 is a single multi-contact plug for connection to a complementary socket 42 on the AED 100, certain of the contacts 44 providing power to the defibrillator and others constituting an external two-way data port. Alternatively the AED 100 could have a protruding multi-contact plug and the SMB 30 a socket to receive it.

The control logic 38 is used to manage the SBM 30 both when it is inserted into the AED 100 and when it is in transit to and later connected to the external computer system 26. Using this mechanism, the embodiment provides a means to solve the problems highlighted above and gives a more satisfactory solution to the requirements for the use of such an AED.

The incorporation of the timing and event synchronisation mechanisms within the SBM 30 inherently means that all data is time stamped. Since the SBM 30 contains its own real-time clock 36 for time reference and also inherently has a power source 32, this real-time clock can continue to function normally as time passes when the SBM 30 is removed from the AED 100.

Furthermore, since the AED 100 can extract setup, operation mode and feature configuration from the SBM memory 34, this effectively means that the SBM 30 becomes the heart of the AED system. The AED 100 in isolation is now no more than a dumb energy transfer and graphic interface device which takes all its command and operational instruction from the SBM 30 once it is plugged into the AED. By providing each AED 100 and each SBM 30 with individual and unique hardwired serially numbered codes, the events, status and usage of each AED and SBM can be easily tracked by the external computer system 26. Thus a particular SMB 30 is not restricted for use with a particular AED 100.

To facilitate data download the external computer system 26 is furnished with a socket (or plug), not shown, the same as that on the AED 100, i.e. complementary to the plug (or socket) 40. Like that between the SMB 30 and AED 100, data communication between the external system 26 and the SBM 30 is two way. This means that as well as retrieving data from any given AED 100 via an SBM 30, the external system 26 can program an SBM. This allows setting of new or different feature settings, an updated time and date stamp for initial setup or for date/time correction, or change the function of the AED as operated with a particular SBM. For instance, any given SBM could be programmed to set the mode of operation to training mode. The next time that that particular SBM was plugged into an AED, that AED would then be in training mode.

Comparing Figs. 1 and 2 it can be seen that by using an SBM 30, there is no longer any need for two connection mechanisms to the AED 100, i.e. one for the memory card and one for the battery pack. Both the battery power connection and the two-way communication/data storage connections have been accomplished simply by plugging in a single SBM. This therefore reduces the number of device case seals that need to be used to seal the device from liquid ingress.

The above embodiment also solves another problem associated with portable battery powered AED. These AEDs require considerable power to enable them to deliver the high-energy shock required to revive the patients heart and restore blood circulation around the body. To provide this power, high density batteries need to be used and the type of batteries that best suit this requirement exhibit a characteristic which makes it very difficult to determine operating lifetime of the device, for any given attached battery pack.

The embodiment allows this power reserve to be easily determined. Since the SBM 30 contains both the battery proper 32 and control logic 38, the control logic can store usage information within the SBM memory 34. As an SBM 30 is repeatedly used, in the same or different AED(s) 100, both the external system and the AED itself can access the usage information. From this usage information and parameters measured from the battery pack (such as voltage), the amount of power left in any given SBM can be accurately calculated and determined. Furthermore, if the SBM battery 32 is rechargeable, the embodiment provides for the external system 26 to recharge the SBM battery and update the usage information such that the AED 100 can appropriately inform the operator the next time the SBM is plugged into an AED.

The invention is not limited to the embodiment described herein which may be modified or varied without departing from the scope of the invention, as defined in the claims.

## Claims

1. A replaceable battery pack (30) for an automatic external defibrillator (100), the battery pack including control logic (38) for controlling the operation of the defibrillator, digital storage means (34) for storing a record of defibrillator events and ECG data, a multi-contact plug or socket (42) for connection to a complementary socket or plug on the defibrillator, wherein certain of the contacts provide power to the defibrillator and others constitute a data port when the battery pack is inserted into an operative position in the defibrillator, and means for connecting the battery pack to an external system (26) for data exchange with, and programming of, the battery pack, **characterised in that** the battery pack (30) further includes a real time clock (36) for time stamping the defibrillator events, and **in that** the means for connecting the battery pack to the external system (26) is the same single plug or socket (42) that is used to connect the battery pack to the defibrillator.

2. A battery pack as claimed in claim 1, wherein the battery pack (30) stores audio data.

3. A battery pack as claimed in claim 1 or 2, wherein the control logic (38) causes the defibrillator (100) to wake-up from a power-off state and perform a self-test function.

4. A battery pack as claimed in claim 3, wherein the control logic (38) causes the defibrillator (100) to perform a self-test function at predetermined time intervals.

5. A battery pack as claimed in claim 4, wherein the results of the self-test are stored to the battery pack (30) for later download.

6. A battery pack as claimed in any preceding claim, wherein the battery pack (30) is configurable for training or test purposes.

7. A battery pack as claimed in any preceding claim, wherein the battery pack (30) stores power usage information.

8. An automatic external defibrillator (100) powered by a replaceable battery pack (30) as claimed in any preceding claim.

## Patentansprüche

1. Ersetzbarer Batteriesatz (30) für einen automatischen externen Defibrillator (100), wobei der Batteriesatz Folgendes umfasst: eine Steuerlogik (38) zur Steuerung des Betriebs des Defibrillators, ein digitales Speichermittel (34) zum Speichern einer Aufzeichnung von Defibrillatorereignissen und EKG-Daten, einen Mehrkontaktstecker oder eine Mehrkontaktbuchse (42) für den Anschluss an eine komplementäre Buchse oder an einen komplementären Stecker an dem Defibrillator, wobei einige der Kontakte den Defibrillator mit Strom versorgen und andere einen Datenport darstellen, wenn der Batteriesatz in einer betriebsfähigen Position in dem Defibrillator eingesetzt ist, sowie Mittel zum Anschließen des Batteriesatzes an ein externes System (26) für den Datenaustausch mit dem Batteriesatz und zum Programmieren des Batteriesatzes, **dadurch gekennzeichnet, dass** der Batteriesatz (30) des Weiteren eine Echtzeituhr (36) umfasst, um die Defibrillatorereignisse mit einem Zeitkennzeichen zu versehen, und dass das Mittel zum Anschließen des Batteriesatzes an das externe System (26) derselbe einzelne Stecker oder dieselbe einzelne Buchse (42) ist, der bzw. die für den Anschluss des Batteriesatzes an den Defibrillator verwendet wird.

2. Batteriesatz nach Anspruch 1, wobei der Batteriesatz (30) Audiodaten speichert.

3. Batteriesatz nach Anspruch 1 oder 2, wobei die Steuerlogik (38) den Defibrillator (100) veranlasst, aus einem ausgeschalteten Zustand aufzuwachen und eine Selbsttestfunktion auszuführen.

4. Batteriesatz nach Anspruch 3, wobei die Steuerlogik (38) den Defibrillator (100) veranlasst, bei vorherbestimmten Zeitintervallen eine Selbsttestfunktion auszuführen.

5. Batteriesatz nach Anspruch 4, wobei die Ergebnisse des Selbsttests zum Herunterladen zu einem späteren Zeitpunkt in dem Batteriesatz (30) gespeichert werden.

6. Batteriesatz nach einem der vorhergehenden Ansprüche, wobei der Batteriesatz (30) für Schulungs- oder Testzwecke konfigurierbar ist.

7. Batteriesatz nach einem der vorhergehenden Ansprüche, wobei der Batteriesatz (30) Stromverbrauchsinformationen speichert.

8. Automatischer externer Defibrillator (100), der von einem ersetzbaren Batteriesatz (30) nach einem der vorhergehenden Ansprüche mit Strom versorgt wird.

## Revendications

1. Bloc de batterie de rechange (30) pour défibrillateur externe automatisé (100), le bloc de batterie comprenant une logique de commande (38) permettant de commander le fonctionnement du défibrillateur, un moyen de stockage numérique (34) permettant de stocker un enregistrement des événements du défibrillateur et des données ECG, une prise ou fiche multicontact (42) à des fins de connexion sur une fiche ou prise complémentaire sur le défibrillateur, dans lequel certains des contacts procurent l'alimentation du défibrillateur et d'autres constituent un port de données quand le bloc de batterie est inséré dans une position de fonctionnement dans le défibrillateur, et un moyen permettant de connecter le bloc de batterie à un système externe (26) à des fins d'échange de données avec le bloc de batterie, et à des fins de programmation de celui-ci, **caractérisé en ce que** le bloc de batterie (30) comprend par ailleurs une horloge temps réel (36) à des fins d'horodatage des événements du défibrillateur, et **en ce que** le moyen permettant de connecter le bloc de batterie au système externe (26) est la même prise ou fiche simple (42) qui est utilisée pour connecter le bloc de batterie au défibrillateur.

2. Bloc de batterie selon la revendication 1, dans lequel le bloc de batterie (30) stocke des données audio.

3. Bloc de batterie selon la revendication 1 ou la revendication 2, dans lequel la logique de commande (38) amène le défibrillateur (100) à se réveiller d'un état de mise hors tension et à réaliser une fonction d'autotest.

4. Bloc de batterie selon la revendication 3, dans lequel la logique de commande (38) amène le défibrillateur (100) à réaliser une fonction d'autotest selon des intervalles prédéterminés.

5. Bloc de batterie selon la revendication 4, dans lequel les résultats de l'autotest sont stockés sur le bloc de batterie (30) à des fins de téléchargement ultérieur.

6. Bloc de batterie selon l'une quelconque des revendications précédentes, dans lequel le bloc de batterie (30) est confgurable à des fins de formation ou de test.

7. Bloc de batterie selon l'une quelconque des revendications précédentes, dans lequel le bloc de batterie (30) stocke des informations se rapportant à la consommation électrique.

8. Défibrillateur externe automatisé (100) alimenté par un bloc de batterie de rechange (30) selon l'une quelconque des revendications précédentes.
